# EUROPEAN PATENT SPECIFICATION

(11) **EP 1 608 237 B1**
(45) Date of publication and mention of the grant of the patent: **20.01.2010**
(21) Application number: 04722828.3
(22) Date of filing: 24.03.2004
(51) Int. Cl.: A23L 1/302, A23L 1/303, A23L 1/305, A23L 1/30, A23L 1/275, A23J 1/14

(54) **POWDEROUS FORMULATIONS OF FAT-SOLUBLE ACTIVE INGREDIENTS**
PULVERFÖRMIGE ZUSAMMENSETZUNGEN VON FETTLÖSLICHEN SUBSTANZEN
FORMULATIONS PULVERULENTES DE PRINCIPES ACTIFS LIPOSOLUBLES

(30) Priority: 03.04.2003 EP 03007677
(43) Date of publication of application: 28.12.2005
(73) Proprietor: DSM IP Assets B.V., 6411 TE Heerlen (NL)
(72) Inventor: FUNDA, Elger, 79639 Grenzach-Wyhlen (DE); HUBER, Torsten, CH-4312 Magden (CH)
(74) Representative: Steck, Melanie
(86) International application number: PCT/EP2004/003110
(87) International publication number: WO 2004/086882

(56) References cited:
- EP-A- 1 106 174
- EP-A- 1 405 572
- WO-A-99/11143
- US-A- 4 892 727
- DATABASE WPI Section Ch, Week 199515 Derwent Publications Ltd., London, GB; Class D13, AN 1995-113373 XP002287568 & RU 2 017 434 C1 (GOLOVCHENKO V I) 15 August 1994 (1994-08-15)
- KING J ET AL: "FUNCTIONAL PROPERTIES OF LUPIN PROTEIN ISOLATES (LUPINUS ALBUS CV MULTOLUPA)" JOURNAL OF FOOD SCIENCE, INSTITUTE OF FOOD TECHNOLOGISTS. CHICAGO, US, vol. 50, no. 1, 1985, pages 82-87, XP002068531 ISSN: 0022-1147

## Description

The present invention is concerned with novel stable powderous formulations comprising a fat-soluble active ingredient, and a process for their preparation. The novel compositions of this invention can be used as additives for food, beverages, animal feeds, cosmetics or drugs to incorporate said fat-soluble ingredients into such application forms.

More specifically, the present invention is concerned with stable powderous formulations comprising a fat-soluble active ingredient in a matrix of a native lupin protein composition.

As used herein, the term "native lupin protein" denotes a lupin protein as it is found in natural products such as lupin seeds and has not been modified by hydrolysis. However, the term "native lupin protein" is understood to include lupin proteins which have undergone post-isoelectric precipitation and are generally known as "restructured" proteins, see international patent application WO 99/11143 and references contained therein.

The term "native lupin protein composition" denotes any composition comprising native lupin protein as obtainable from natural lupin protein sources. Examples of such native lupin protein compositions are lupin protein concentrates, which have a protein content of 60 % up to 90 % by weight (hereinafter : wt.-%), generally from 50-96 wt.-%, typically about 65-70 wt.-% of protein; and lupin protein isolates, which term is generally used in the art to define protein preparations containing more than about 90 wt.-% of protein. The residual constituents (4-50 wt.-%) of such concentrates and isolates are, besides water and oil, primarily plant fibers.

For the purpose of the present invention, lupin concentrates having a protein content of about 60- 90 wt.-%, isolates having a protein content of more than 90 wt.-%, and flours having a protein content of about 40-60 wt.-%,
are preferred. As a source for the protein compositions all known lupin varieties, such as
Lupine Angustifolius, Lupine Albus oder Lupine Luteus can be used. However, protein compositions derived from Lupine Angustifolius and Lupine Albus are preferred.

The term "fat-soluble active ingredient" as used herein denotes any physiologically active ingredient that is soluble in lipids and insoluble or sparingly soluble in water. Examples of such fat-soluble active ingredients are fat-soluble vitamins, viz., vitamin A, D, E and K and derivatives thereof such as vitamin A esters, e.g. vitamin A acetate and palmiate, and vitamin E esters, e.g. tocopherol acetate; carotenoids and carotinoid derivatives, e.g., are α- or β-carotene, 8'-apo-β-carotenal, 8'-apo-β-carotenoic acid esters such as the ethyl ester, canthaxanthin, astaxanthin, astaxanthin esters, lycopene, lutein, zeaxanthin or crocetin and their derivatives; polyunsaturated fatty acids, e.g. eicosapentaenoic acid, docosahexaenoic acid, arachidonic acid and and γ-linolenic acid and/or ethylester. The fat-soluble active ingredient may be present in the formulation in an amount of from about 0.1 wt.-% to about 80 wt.-%, especially from about 0.5 wt.-% to about 60 wt.-%, based on the total weight of the composition.

In a preferred aspect of the invention, the novel formulations additionally contain a reducing sugar, e.g. glucose, fructose, or xylose in an amount of from about 0.1 wt.-% to about 70 wt.-%, especially from about 1.0 to about 10 wt.-%, based on the total weight of the composition.

Such formulations can be submitted to heat-treatment to cause cross-linking of the sugar with the protein in a Maillard type reaction. Crosslinking can be also achieved by treatment with enzymes like transglutaminase in a manner know per se, see, e.g., US 5,156,956. The cross-linked formulations have been found to exhibit increased stability.

In accordance with the invention, the novel formulations can be obtained by a process which comprises preparing an aqueous emulsion of the fat-soluble active ingredient and the native lupin protein composition, if desired, adding a reducing sugar, converting the emulsion into a dry powder and, if a reducing sugar was added, submitting the dry powder to cross-linking the sugar with the protein by heat treatment or by treatment with a cross-linking enzyme.

Suitably, in a first step of the process of the invention, the protein composition is dispersed in water. Thereafter, the fat-soluble active ingredient is emulsified, suitably in liquid state, i.e. with adequate warming and/or as a solution in an appropriate solvent, into the aqueous dispersion of the protein. Alternatively a suspension of the solid active may be produced by appropriate procedures like milling. The emulsion is then, optionally after removal of excess solvent, spray-dried. The spray-drying can effected be using conventional technology of spray-drying, spray drying in combination with fluidized-bed granulation (the latter technique commonly known as fluidized spray drying or FSD), or by a powder-catch technique where sprayed emulsion droplets are caught in a bed of an absorbant such as starch or calcium silicate and subsequently dried.
In still another aspect of the invention, the novel formulations may additionally contain other proteins or hydrolyzed proteins that act as protective colloids, e.g. soy proteins or, hydrolyzed soy proteins. Such additional proteins may be present in the formulations of the invention in an amount of from 10-50 wt.-% based on the total amount of protein in the formulation.

Finally, in a still further aspect, the present invention is concerned with food, beverages, animal feeds, cosmetics and drugs which comprise the novel formulations of the present invention.

The novel formulations of this invention may further contain adjuvants and/or excipients such as one or more of a mono- di-, oligo- or polysaccharide, a triglyceride, a water-soluble antioxidant, a fat-soluble antioxidant, silicic acid, Ca-silicate, Ca-carbonate and water.

Examples of mono- and disaccharides which may be present in the formulations of the present invention are saccharose, invert sugar, glucose, fructose, lactose and maltose. Examples of oligo- or polysaccharides which may be present in the compositions of the present invention are starch, modified starch and starch hydrolysates, such as dextrins and maltodextrins, especially such in the range of 5-65 dextrose equivalents (hereinafter: DE) and glucose syrup, especially such in the range of 20-95 DE. The term "dextrose equivalent" (DE) denotes the degree ofhydrolysation and is measure for the amount of reducing sugar calculated as D-glucose based on dry weight. Native starch has DE close to 0 while glucose has a DE = 100.

The triglyceride is suitably a vegetable oil or fat, such as corn oil, sunflower oil, soybean oil, safflower oil, rape seed oil, arachis oil, palm oil, palm kernel oil, cotton seed oil or cocos oil.

The water-soluble antioxidant may be ascorbic acid and salts thereof, e.g., sodium ascorbate, and the like. The fat-soluble antioxidant may be a tocopherol, e.g., dl-α-tocopherol (i.e., synthetic tocopherol), d-α-tocopherol (i.e., natural tocopherol), β- and γ-tocopherol and mixtures thereof; ascorbic acid esters of fatty acids such as ascorbyl palmitate or stearate; butyl hydroxy toluene (BHT); butyl hydroxy anisol (BHA); propyl gallate; or t-butyl hydroxy quinoline; or 6-ethoxy-1,2-dihydroxy-2,2,4-trimethylquinoline (EMQ).

The following Examples illustrate the invention further.

### Example 1

### Preparation of a powderous vitamin A formulation:

62.4 g of lupin protein isolate from Lup. Angustifolius (protein content 96.2%) and 10.9 g of glycerol were added to 230 ml of water. The mixture was warmed to 60°C until dissolution occurred. To this solution, 12.3 g of fructose were added and the pH of the solution was adjusted to 6.5 ± 0.2. Thereafter, 49.3 g of vitamin A acetate (2,1 x 10⁶ IE vitamin A /g stabilized with Ethoxyquin) were emulsified into the matrix solution whereupon the mixture was stirred for 60 minutes at 60°C. The inner phase of the emulsion then exhibited a mean particle size of about 580 nm. The emulsion was then diluted with ca. 25 ml of water and about 300 g of the emulsion was sprayed in a spraying pan in a bed of Ca-silicate at about 5° C by means of a rotating spraying nozzle. The so-obtained beadlets werde separated from excess Ca-silicate by sieving and dried. There were obtained ca. 100 g of dry powder having a vitamin A content of ca. 850'000 IEA/g.

### Example 2

### Thermal cross-linking:

The vitamin A dry powder obtained in Example 1 is stirred at a temperature of 135°C for 35 minutes. The so-obtained product was insoluble in hot water and had a vitamin A content of ca. 570'000 IEA/g.

### Example 3

### Preparation of an ethyl apo-carotenoate dry powder :

a) 16 g of lupin protein isolate from Lup. Angustifolius (protein content 96.2%) were dissolved in 130 ml of water at 50° C. To this solution, 1.6 g of ascorbylpalmitate were added and the pH of the solution was adjusted to 7.5 ± 0.2 by the addition of 20 wt.-% sodium hydroxide solution.
b) 9 g of ethyl β-apo-8'-carotenoate, 5.5 g of corn oil and 0.6 g of Ethoxyquin were dissolved in 50 ml of chloroform.
c) The ethyl β-apo-8'-carotenoate solution obtained in paragraph b) was emulsified during 30 minutes at 45° C into the solution obtained in paragraph a). The inner phase of the emulsion then exhibited a mean particle size of about 280 nm. The chloroform was evaporated at 50° C under reduced pressure and the emulsion was spray-dried in analogy to the procedure of Example 1 in a bed of starch. There were obtained 42 g of dry powder having an ethyl β-apo-8'-carotenoate content of 11,4 wt.- %.

## Claims

1. Stable powderous formulations comprising a fat-soluble active ingredient in a matrix of a native lupin protein composition wherein the protein is cross-linked.

2. Formulations according to claim 1, wherein the lupin protein composition is a lupin protein isolate having a protein content of more than 90 wt.-%.

3. Formulations according to claim 1, wherein the lupin protein composition is a lupin protein concentrate having a protein content of 60-90 wt.-%.

4. Formulations according to claim 1, wherein the lupin protein composition is a lupin protein flour having a protein content of 40-60 wt.-%.

5. Formulations according to claim 1, comprising mixtures of native lupin protein compositions as defined in claims 2-4.

6. Formulations according to claim 1, wherein the fat-soluble active ingredient is vitamin A, D, E or K, or a carotenoid, or a polyunsaturated fatty acid, or esters thereof,or mixtures thereof.

7. Formulations according to claim 1, wherein the fat-soluble active ingredient is a plant or animal oil or fat, particularly sunflower oil, palm oil or corn oil.

8. Formulations according to claim 1, comprising additionally a reducing sugar, particularly glucose, fructose, or xylose.

9. Food, beverages, animal feeds, cosmetics or drugs comprising a formulation according to any one of claims 1-8.

10. Process for the preparation of formulations according to any one of claims 1 - 8, which comprises preparing an aqueous emulsion of the fat-soluble active ingredient and the native lupin protein composition, if desired, adding a reducing sugar, converting the emulsion into a dry powder, and submitting the dry powder to cross-linking the protein by heat treatment or by treatment with a cross-linking enzyme.

11. A process according to claim 10 wherein a reducing sugar is added and the composition is submitted to crosslinking by heating.

12. A process according to claim 10 wherein the composition is submitted to crosslinking by treatment with a cross-linking enzyme, particularly transglutaminase.

## Patentansprüche

1. Stabile pulverförmige Zusammensetzungen, die einen fettlösliche Wirkstoff in einer Matrix aus einer nativen Lupinenproteinzusammensetzung umfassen, wobei das Protein vernetzt ist.

2. Formulierungen nach Anspruch 1, wobei es sich bei der Lupinenproteinzusammensetzung um ein Lupinenproteinisolat mit einem Proteingehalt von mehr als 90 Gew.-% handelt.

3. Formulierungen nach Anspruch 1, wobei es sich bei der Lupinenproteinzusammensetzung um ein Lupinenproteinkonzentrat mit einem Proteingehalt von 60-90 Gew.-% handelt.

4. Formulierungen nach Anspruch 1, wobei es sich bei der Lupinenproteinzusammensetzung um ein Lupinenproteinmehl mit einem Proteingehalt von 40-60 Gew.-% handelt.

5. Formulierungen nach Anspruch 1, die Mischungen von nativen Lupinenproteinzusammensetzungen wie in den Ansprüchen 2-4 definiert umfassen.

6. Formulierungen nach Anspruch 1, wobei es sich bei dem fettlöslichen Wirkstoff um Vitamin A, D, E oder K oder um ein Karotinoid oder um eine mehrfach ungesättigte Fettsäure oder Ester davon oder Mischungen davon handelt.

7. Formulierungen nach Anspruch 1, wobei es sich bei dem fettlöslichen Wirkstoff um ein pflanzliches oder tierisches Öl oder Fett, insbesondere Sonnenblumenöl, Palmöl oder Maiskeimöl, handelt.

8. Formulierungen nach Anspruch 1, die zusätzlich einen reduzierenden Zucker, insbesondere Glucose, Fructose oder Xylose, umfassen.

9. Nahrungsmittel, Getränke, Tierfutter, Kosmetika oder Arzneimittel, die eine Formulierung nach einem der Ansprüche 1-8 umfassen.

10. Verfahren zur Herstellung von Formulierungen nach einem der Ansprüche 1-8, bei dem man eine wäßrige Emulsion des fettlöslichen Wirkstoffs und der nativen Lupinenproteinzusammensetzung herstellt, gewünschtenfalls einen reduzierenden Zucker zufügt, die Emulsion in ein Trockenpulver überführt, und mit dem Trockenpulver ein Vernetzen des Proteins durch Hitzebehandlung oder durch Behandlung mit einem vernetzenden Enzym durchführt.

11. Verfahren nach Anspruch 10, wobei man mit einem reduzierenden Zucker versetzt und man mit der Zusammensetzung ein Vernetzen durch Erhitzen durchführt.

12. Verfahren nach Anspruch 10, wobei man mit der Zusammensetzung ein Vernetzen durch Behandlung mit einem vernetzenden Enzym, insbesondere Transglutaminase, durchführt.

## Revendications

1. Formulations en poudre stables comprenant un principe actif liposoluble dans une matrice d'une composition de protéine de lupin native, la protéine étant réticulée.

2. Formulations selon la revendication 1, **caractérisées en ce que** la composition de protéine de lupin est un isolat de protéine de lupin ayant une teneur en protéine supérieure à 90 % en poids.

3. Formulations selon la revendication 1, **caractérisées en ce que** la composition de protéine de lupin est un concentré de protéine de lupin ayant une teneur en protéine de 60-90 % en poids.

4. Formulations selon la revendication 1, **caractérisées en ce que** la composition de protéine de lupin est une farine de protéine de lupin ayant une teneur en protéine de 40-60 % en poids.

5. Formulations selon la revendication 1, comprenant des mélanges de compositions de protéine de lupin native telles que définies dans les revendications 2-4.

6. Formulations selon la revendication 1, **caractérisées en ce que** le principe actif liposoluble est la vitamine A, D, E ou K, ou un caroténoïde, ou un acide gras polyinsaturé, ou des esters de celui-ci, ou des mélanges de ceux-ci.

7. Formulations selon la revendication 1, **caractérisées en ce que** le principe actif liposoluble est une huile ou une graisse végétale ou animale, en particulier l'huile de tournesol, l'huile de palme ou l'huile de maïs.

8. Formulations selon la revendication 1, comprenant en outre un sucre réducteur, en particulier le glucose, le fructose ou le xylose.

9. Aliment pour l'homme, boissons, aliments pour animaux, produits cosmétiques ou médicaments comprenant une formulation selon l'une quelconque des revendications 1-8.

10. Procédé de préparation de formulations selon l'une quelconque des revendications 1-8, **caractérisé en ce qu'**il comprend la préparation d'une émulsion aqueuse du principe actif liposoluble et de la composition de protéine de lupin native, si on le souhaite, l'addition d'un sucre réducteur, la transformation de l'émulsion en poudre sèche, et la soumission de la poudre sèche à une réticulation de la protéine par traitement à la chaleur ou par traitement avec une enzyme de réticulation.

11. Procédé selon la revendication 10, **caractérisé en ce que** le sucre réducteur est ajouté et la composition est soumise à une réticulation par chauffage.

12. Procédé selon la revendication 10, **caractérisé en ce que** la composition est soumise à une réticulation par traitement avec une enzyme de réticulation, en particulier la transglutaminase.
